# EUROPEAN PATENT APPLICATION

(11) **EP 1 184 466 A2**
(43) Date of publication of application: **06.03.2002**
(21) Application number: 01307203.8
(22) Date of filing: 24.08.2001
(51) Int. Cl.: C12Q 1/68

(54) **Target nucleic acid enrichment and amplification for array analysis**

(30) Priority: 26.08.2000 US 228253 P; 19.01.2001 US 766212
(71) Applicant: Affymetrix, Inc., Santa Clara, CA 95051 (US)
(72) Inventor: Dong, Shoulian, San Jose, California 95132 (US); Kennedy, Giulia, San Francisco, California 94116 (US); McAllister, Linda, San Francisco, California 94127 (US); Su, Xing, Cupertino, California 95014 (US)
(74) Representative: Kirkham, Nicholas Andrew

(57) **Abstract**

The presently claimed invention provides methods and kits for isolating and amplifying a specific target sequence from within a nucleic acid population. The presently claimed invention provides selection probes which are complementary to at least a portion of said target sequence and mechanisms for isolating the target-probe complexes from the rest of the nucleic acid population.

## Description

The invention relates to enrichment and amplification of specific nucleic acid sequences and is well suited for the enrichment and amplification of both long and short DNA sequences. In one embodiment, the invention relates to enrichment and amplification of whole or partial chromosomes for the purpose of further analysis. In another embodiment, the invention relates to the enrichment and amplification of known or putative polymorphic regions. As such the invention relates to the fields of molecular biology and genetics.

Methods of amplifying target DNA sequences tend to be costly and complicated, often requiring large numbers of specific primer sequences which must be synthesized for each experiment. The cost and difficulty are dramatically increased with the length of the target sequences. Novel, more cost effective and less complex methods for enriching and amplifying specific nucleic acid sequences including polymorphic regions, chromosomal regions and whole chromosomes are desirable.

The currently claimed invention provides novel methods for the reproducible enrichment and amplification of specific nucleic acid target sequences from a nucleic acid population. The invention further provides kits and apparatus for the enrichment and amplification of specific nucleic acid target sequences from a nucleic acid population.

In a first embodiment, the currently claimed invention provides a method of reproducibly obtaining a population of target fragments wherein each of said target fragments comprises a region of interest. The method comprises fragmenting the nucleic acid population and exposing the fragments to selection probes. The selection probes comprise a separation element and region which is complementary to the region of interest. Complementary fragments and selection probes are allowed to hybridize, forming probe-target complexes. The probe-target complexes are then separated from any non-hybridized fragments via the separation element. The probe-target complexes are then denatured producing an enriched population of target fragments and selection probes. If desired, the selection probes may be removed via the separation element.

In a second embodiment, the presently claimed invention provides a method of analyzing a nucleic acid sample. The method comprises fragmenting the nucleic acid population and exposing the fragments to selection probes. The selection probes comprise a separation element and region which is complementary to the region of interest. Complementary fragments and selection probes are hybridized, forming probe-target complexes. The probe-target complexes are then separated from any non-hybridized fragments via the separation element. The probe-target complexes are then denatured, producing an enriched population of target fragments and selection probes. If desired, the selection probes may be removed via the separation element. The target fragments are then amplified and hybridized to a nucleic acid array.

In a third embodiment, the presently claimed invention provides a kit for reproducibly obtaining a population of target fragments wherein each of the target fragments comprise a region of interest. The kit comprises a population of cells comprising cloning vectors. The cloning vectors comprise nucleic acid insert sequences which are complementary to at least a portion of said region of interest. The kit further comprises a plurality of separation elements which are capable of being attached to fragments from the cloning vectors.

In a fourth embodiment, the presently claimed invention provides a method of genotyping an individual. The method comprises obtaining a sample of genomic DNA from an individual, fragmenting the sample to form genomic fragments and exposing the genomic fragments to selection probes. The selection probes comprise a separation element and a region which is complementary to a region of DNA which is known or believed to be adjacent to a polymorphic site. The genomic fragments are exposed to the selection probes under suitable conditions to allow for hybridization, forming probe-target complexes. The probe-target complexes are then separated from any non-hybridized fragments via the separation element. Once separated from the non-hybridized fragments, the probe-target complexes are denatured to produce target fragments and selection probes. The target sequences are then amplified and hybridized to an array of immobilized probes capable of interrogating the polymorphic site. The immobilized probes are attached to a solid support at known locations. Hybridization between the immobilized probe and the target sequence is then detected. The identity of the polymorphic base is determined from the hybridization information.

In a fifth embodiment, the presently claimed invention provides an isolated fragment comprising a region of interest. The isolated fragment is obtained by fragmenting a nucleic acid population and exposing the fragments to a selection probe. The selection probe comprises a separation element and a region which is complementary to the region of interest. The selection probe hybridizes to a complementary fragment, forming a probe-target complex. The probe-target complex is then separated from any non-hybridized fragments via the separation element. The probe-target complex is then denatured to produce a target fragment and a selection probe. The target fragment is then isolated from the selection probe.

In a sixth embodiment, the presently claimed invention provides a method for reproducibly obtaining target sequences for further analysis. The method comprises fragmenting a nucleic acid sample and hybridizing the fragments to a nucleic acid array comprising immobilized selection probes, each selection probe being designed to be complementary to at least a portion of one of the target fragments. Any non-hybridized fragments are then removed. The hybridized fragments are then eluted from the array, producing a pool of target sequences.

In a seventh embodiment, the presently claimed invention provides an apparatus for reproducibly obtaining a population of target fragments comprising an array of selection probes immobilized to a solid support, wherein each of the selection probes is designed to be complementary to at least a portion of one of the target fragments.

### BRIEF DESCRIPTION OF THE DRAWINGS

Figure 1 is a schematic illustration of a method for isolating a target sequence from a nucleic acid population.
Figure 2 is a schematic illustration of a method for obtaining selection probes from a cloning vector.
Figure 3 is a schematic illustration of a method for amplifying a target sequence from a nucleic acid population.
Figure 4 is a schematic illustration of one method of attaching adapter sequences to nucleic acid fragments employing tag sequences and tailing.
Figure 5 is a schematic illustration of a method of isolating specific polymorphic sites in a nucleic acid population.
Figure 6 is the scanned image of the initial hybridization of a sample to a nucleic acid array.
Figure 7 is the scanned image of the array in Figure 6 after the hybridized sample was eluted from the array.
Figure 8 is a scanned image of the hybridization of the re-amplified eluted fragments from Figure 6 to a second array.
Figure 9 is a scanned image of the initial hybridization of a sample containing 10⁷ copies.
Figure 10 is the scanned image of the re-amplified eluted fragments from Figure 9 hybridized to a second array.
Figure 11 is the scanned image of the initial hybridization of a sample containing 10⁵ copies.
Figure 12 is the scanned image of the re-amplified eluted fragments from Figure 11 hybridized to a second array.
Figure 13 shows scanned images of bead enriched targets from HGE and Total Genomic DNA.
Figure 14 shows the scanned images of BAC3 DNA before and after PCR amplification.

### (A) Definitions

The terms "nucleic acid," "sequence" or "nucleic acid sequence" refer to a deoxyribonucleotide or ribonucleotide polymer in either single-or double-stranded form, and unless otherwise limited, would encompass analogs of natural nucleotides that can function in a similar manner as naturally occurring nucleotide. Nucleic acids may be derived from a variety of sources including, but not limited to, natural or naturally occurring nucleic acids or mimetics thereof, clones, synthesis in solution or solid phase synthesis.

An "oligonucleotide" or "polynucleotide" is a nucleic acid ranging from at least 2, preferable at least 8, and more preferably at least 20 nucleotides in length or a compound that specifically hybridizes to a polynucleotide. Polynucleotides of the present invention include sequences of deoxyribonucleic acid (DNA) or ribonucleic acid (RNA) or mimetics thereof which may be isolated from natural sources, recombinantly produced or artificially synthesized. A further example of a polynucleotide of the present invention may be a peptide nucleic acid (PNA). The invention also encompasses situations in which there is a nontraditional base pairing such as Hoogsteen base pairing which has been identified in certain tRNA molecules and postulated to exist in a triple helix. "Polynucleotide" and "oligonucleotide" are used interchangeably in this application.

The terms "fragment," "segment," or "DNA segment" refer to a portion of a larger DNA polynucleotide or DNA. A polynucleotide, for example, can be broken up, or fragmented into, a plurality of segments.

"Genome" designates or denotes the complete, single-copy set of genetic instructions for an organism as coded into the DNA of the organism. A genome may be multi-chromosomal such that the DNA is cellularly distributed among a plurality of individual chromosomes. For example, in human there are 22 pairs of chromosomes plus a gender associated XX or XY pair.

The term "chromosome" refers to the heredity-bearing gene carrier of a living cell which is derived from chromatin and which comprises DNA and protein components (especially histones). The conventional internationally recognized individual human genome chromosome numbering system is employed herein. The size of an individual chromosome can vary from one type to another with a given multi-chromosomal genome and from one genome to another. In the case of the human genome, the entire DNA mass of a given chromosome is usually greater than about 100,000,000 bp. For example, the size of the entire human genome is about 3 x 10⁹ bp. The largest chromosome, chromosome no. 1, contains about 2.4 x 10⁸ bp while the smallest chromosome, chromosome no. 22, contains about 5.3 x 10⁷ bp.

A "chromosomal region" is a portion of a chromosome. The actual physical size or extent of any individual chromosomal region can vary greatly. The term "region" is not necessarily definitive of a particular one (or more) genes because a region need not take into specific account the particular coding segments (exons) of an individual gene.

The term "target sequence" refers to a nucleic acid (often derived from a biological sample), to which a probe is designed to specifically hybridize. The target nucleic acid comprises a sequence that is complementary to the nucleic acid sequence of the corresponding probe. The term target nucleic acid may refer to the specific subsequence of a larger nucleic acid to which the probe is directed or to the overall sequence. The target sequence may or may not be of biological significance. Typically, though not always, it is the significance of the target sequence which is being studied in a particular experiment. As non-limiting examples, target sequences may include regions of genomic DNA which are believed to contain one or more polymorphic sites, DNA encoding or believed to encode genes or portions of genes of known or unknown function, DNA encoding or believed to encode proteins or portions of proteins of known or unknown function, etc.

As used herein a "probe" is defined as a nucleic acid, such as an oligonucleotide, capable of binding to a target nucleic acid of complementary sequence through one or more types of chemical bonds, usually through complementary base pairing, usually through hydrogen bond formation. As used herein, a probe may include natural (*i.e.* A, G, U, C, or T) or modified bases (7-deazaguanosine, inosine, *etc.).* In addition, the bases in probes may be joined by a linkage other than a phosphodiester bond, so long as it does not interfere with hybridization. Thus, probes may be peptide nucleic acids in which the constituent bases are joined by peptide bonds rather than phosphodiester linkages.

A "cloning vector" is typically a DNA molecule originating from a virus, a plasmid, or the cell of a higher organism into which another DNA fragment of appropriate size can be integrated without loss of the vector's capacity for self replication. Vectors are often used to introduce foreign DNA into host cells where the DNA can be reproduced in large quantities. Examples are plasmids, cosmids, yeast artificial chromosomes (YACs) and bacteria artificial chromosomes (BACs). Vectors may be recombinant molecules containing DNA sequences from several sources.

The present invention relies on many patents, applications and other references for details known to those of the art. Therefore, when a patent, application or other reference is cited or repeated below, it should be understood that it is incorporated by reference in its entirety for all purposes as well as for the proposition that is recited.

### (B) General

In one embodiment, the currently claimed invention provides a method of enriching for specific nucleic acid target sequences in a nucleic acid population. In this embodiment, the currently claimed invention provides a method of reproducibly obtaining a population of target fragments wherein each of said target fragments comprises a region of interest. The method comprises fragmenting the nucleic acid population and exposing the fragments to selection probes. The selection probes comprise a separation element and region which is complementary to the region of interest. Complementary fragments and selection probes are allowed to hybridize, forming probe-target complexes. The probe-target complexes are then separated from any non-hybridized fragments via the separation element. The probe-target complexes are then denatured producing an enriched population of target fragments and selection probes. If desired, the selection probes may be removed via the separation element.

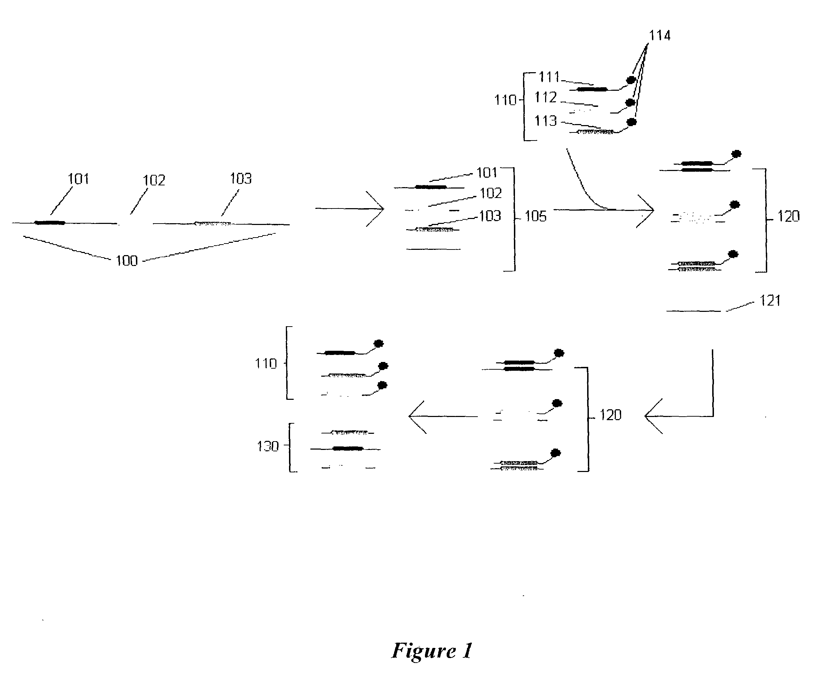

Figure 1 depicts a schematic illustration of the general steps of a preferred embodiment of the currently claimed invention. First, a nucleic acid population 100 containing a number of regions of interest 101, 102 and 103 is obtained. The nucleic acid population 100 is fragmented to produce fragments 105. A subpopulation of these fragments contain the regions of interest. The fragments are then exposed to selection probes 110. Each selection probes comprises a selection element 114 and a complementary segment which is capable of hybridizing to at least a portion of one of the regions of interest, 111, 112 and 113. In Figure 1, complementary segment 111 is capable of hybridizing to region of interest 101, complementary segment 112 is capable of hybridizing to region of interest 102 and complementary segment 113 is capable of hybridizing to region of interest 103. The selection probes are allowed to specifically hybridize to the fragments, producing target-probe complexes 120. The selection probes are designed such that those fragments which do not contain a region of interest will not hybridize to the selection probes (see non-hybridized fragment 121). The target-probe complexes are then isolated from the population via the separation element. Thereafter, if desired, the fragments containing the regions of interest 101, 102, 103 (collectively "target sequences") may be separated and isolated from the selection probes 110 producing isolated target sequences 130. As an option, these target sequences may be amplified and/or analyzed using any variety of methods.

The nucleic acid population may be a sample derived from any number of sources including genomic DNA, cDNAs, pools of fragments, cloned sequences, etc. Any suitable biological sample can be used for assay of genomic DNA. Convenient suitable tissue samples include whole blood, semen, saliva, tears, urine, fecal material, sweat, buccal, skin and hair. Pure red blood cells are not suitable. As those skilled in the art will appreciate, for assays of cDNA or mRNA, the tissue sample must be obtained from an organ in which the target nucleic acid is expressed, e.g., the liver for a target nucleic acid of a cytochrome P450.

The region of interest may be of any length and may comprise any sequence. For example, the region of interest may be a polymorphic site and comprise a single nucleotide base or may be an entire chromosome and comprise a million or more nucleotide bases. The region of interest may contain a variable region. The variable region may or may not be associated with a particular phenotype.

Any known method of fragmentation may be employed. Various methods of fragmenting nucleic acids will be known to those of skill in the art. These methods may be, for example, either chemical or physical in nature. Chemical fragmentation may include partial degradation with a DNAse, partial depurination with acid, restriction enzymes or other enzymes which cleave nucleic acid at known or unknown locations. Physical fragmentation methods may involve subjecting the nucleic acid to a high shear rate. High shear rates may be produced, for example, by moving nucleic acid through a chamber or channel with pits or spikes, or forcing the nucleic sample through a restricted size flow passage, e.g. an aperture having a cross sectional dimension in the micron or submicron scale. Combinations of physical and chemical fragmentation methods may likewise be employed such as fragmentation by heat and ion-mediated hydrolysis.

Those of skill in the art will be familiar with the digestion of nucleic acids with restriction enzymes. In a preferred embodiment of the invention, particularly when genomic DNA is used as the sample source, a combination of restriction enzymes is used, as specific combinations of restrictions enzymes may result in a larger percentage of genomic DNA fragments of suitable length for amplification.

As one of skill in the art will appreciate, longer DNA fragments are more difficult to amplify with high fidelity. A specific restriction enzyme will typically cut the DNA at a given recognition sequence, and that recognition sequence statistically appears in the genomic DNA every X number of base pairs, where X varies with the length of the given recognition sequence (i.e. restrictions enzymes which have four base recognition site will cut more frequently than restriction enzymes with a six or eight base recognition site). Thus, the combination of restrictions enzymes to be used may be altered to produce fragments in a desired range of sizes.

It may be desirable to denature the target sequence prior to exposure to the selection probes. Denaturation may take place before or after fragmentation. Whether to denature before or after fragmentation may depend on the method of fragmentation employed. Furthermore, if adapter sequences are to be attached, as described below, it may be preferable to attach double stranded adapter sequences to double stranded fragments prior to denaturation. Alternatively some methods of adapter attachment may require a single-stranded template.

Those of skill in the art will be familiar with conditions required to allow for suitable hybridization between the target sequence and the selection probe. See for example, Maniatis, et al., "Molecular Cloning: A Laboratory Manual," 2^{nd} Ed. Cold Spring Harbor Laboratory Press, Cold Spring Harbor, New York (1989) ("Maniatis et al.")

The selection probes may be obtained from any number of sources. In one embodiment, the selection probes are comprised of fragments of a larger nucleic acid sequence. Alternatively, the selection probe may be comprised of a short nucleic acid sequence which has been synthesized or isolated from a biological or non-biological source. The separation element may be attached to the selection probe through chemical or other means or may be an inherent part of the selection probe. If the selection probe is synthesized de novo, the separation element may be synthesized with the selection probe, attached to the selection probe during synthesis, or attached during a post-synthesis step.

The length of the selection probe may vary. The length need only be long enough to hybridize specifically to the desired region. The selection probe will typically be from 17 to 250 bases and more specifically from 20 to 35 bases.

The complementary regions of each selection probe may be different, as in Figure 1, or the selection probes may all include identical complementary regions. The complementary regions may comprise any sequence which will selectively hybridize to at least a portion of the region of interest. Thus, if the region of interest is very long, such as a partial chromosomal regions, the complementary region need not be exactly complementary to the chromosomal region, it need only be capable of selectively hybridizing to the chromosomal region with a higher affinity than any of the other sequences in the nucleic acid population. The presently claimed invention does not require that each of the selection probes hybridize to a nucleic acid fragment. It is acceptable for only a small portion of the pool of selection probes to hybridize to nucleic acid fragments from a given sample. In this manner, generic selection probe pools may be created which can be used for a variety of different samples, some selection probes may be capable of hybridizing to nucleic acid fragments from a number of different samples while others are sample specific. For example, a selection probe pool may contain probes with complementary regions for sequences which are known to be conserved between two or more different species. The same selection probe pool may also contain complementary regions for species-specific sequences. This same probe pool could then be used to enrich samples provided from one or more of the species.

In one embodiment, the selection probes are obtained by fragmenting long interrogation sequences and then attaching each fragment to a separation element. This produces a number of selection probes which in combination can interrogate long sequences including, for example, an entire coding region, a gene, an operon, a partial chromosome, an entire chromosome, or an entire genome. These long DNA sequences may be between tens and millions of nucleotides in length. Once fragmented into segments of appropriate length, the segments may be attached to separation elements to form selection probes.

In one embodiment, prior to fragmentation, the long DNA sequences are cloned into vectors. This embodiment is preferred when the interrogation sequences are particularly long such as a partial or entire chromosome. An advantage of this embodiment is that cloning vectors containing chromosomal regions or whole chromosomes are widely available. While known methods can be used to insert DNA sequences into cloning vectors (see, for example, Maniatis et al., which is incorporated by reference above), cloning vectors containing whole chromosomes and / or chromosomal regions can be ordered from any number of sources, including, for example, Research Genetics (Huntsville, AL). In a preferred embodiment the cloning vectors are bacterial artificial chromosomes (BACs) or yeast artificial chromosomes (YACs). BACs and YACs are able to carry very large sequences, such as large chromosomal regions, or even whole chromosomes. Advantageously, only a very small portion of the BAC or YAC is made up of endogenous backbone DNA, thus the number of fragments containing endogenous bacterial or yeast DNA is not enough to interfere with the experiment. A further advantage of this embodiment is that cloning vectors comprising the desired interrogation sequence can be maintained in a population of cells.

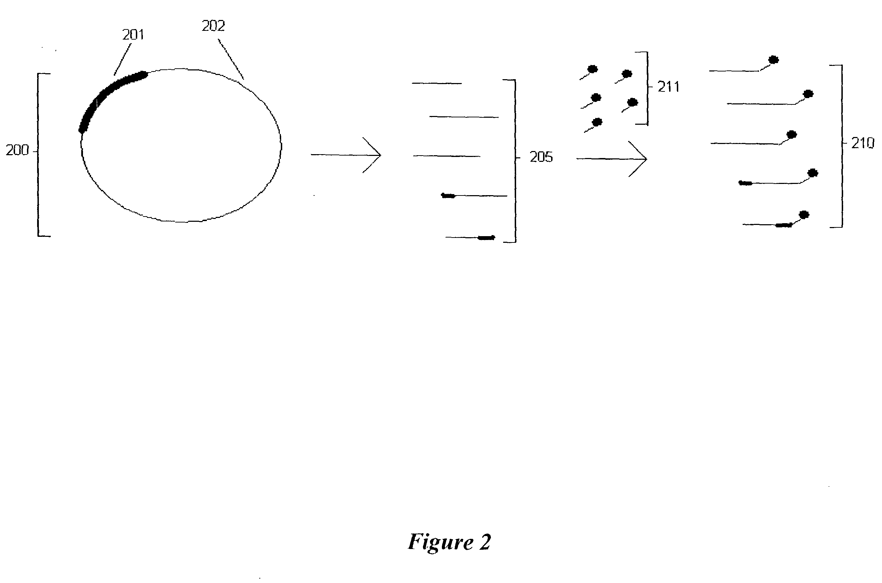

In Figure 2, a cloning vector 201 comprises backbone 210 and chromosomal sequence 202. The cloning vector is first fragmented and the vector fragments 205 are then bound to a separation element 211 producing selection probes 210.

The separation element may take advantage of any mechanism by which target-probe complexes may be isolated from the nucleic acid population. For example, the separation element may be capable of binding a solid support such as a bead, fiber or array. The separation element may comprise, for example, a specific nucleic acid sequence, an antibody capable of binding a receptor, a ligand capable of binding a receptor, an antigen capable of binding an antibody, an antiidiotypic antibody capable of binding an antibody, an antibody capable of binding an antiidiotypic antibody, a haptenic group capable of binding an antibody, an antibody for a haptenic group capable of binding a haptenic group, an enzyme capable of binding an enzyme inhibitor or an enzyme inhibitor capable of binding an enzyme, etc. In the example wherein the separation element is a nucleic acid sequence, the sequence which is complementary to the separation element may be bound to a solid support. Furthermore, the separation element may be the solid support itself, with the complementary segment being directly immobilized to the solid support.

In a preferred embodiment, the separation element comprises a solid support to which the complementary sequence is bound. This solid support may include a wide variety of supports, including, but not limited to beads, glass slides, silicon, quartz, and fibers. Solid supports and methods of attaching nucleic acid sequences to solid supports will be well known to those of skill in the art and are described in, for example US Patent Nos. 5,800,992, 6,040,193, 5,143,854, 5,384,261, 5,405,783, 5,412,087, 5,424,186, 5,252,743, 5,451,683, 5,482,867, 5,510,270, 5,744,305, and 5,837,832.

In a preferred embodiment, a selection array is used, wherein an array of selection probes is immobilized to a solid support. In this embodiment, the probes may be immobilized at known or unknown locations. Methods for making and using arrays which are suitable for this embodiment are described in, for example US Patents 5,143,854, 5,242,979, 5,252,743, 5,324,663, 5,384,261, 5,405,783, 5,412,087, 5,424,186, 5,445,934, 5,451,683, 5,482,867, 5,489,678, 5,491,074, 5,510,270, 5,527,681, 5,550,215, 5,571,639, 5,593,839, 5,599,695, 5,624,711, 5,631,734, 5,677,195, 5,744,101, 5,744,305, 5,753,788, 5,770,456, 5,831,070,5,856,011, 5,858,695, 5,861,242, 5,871,928, 5,874,219, 5,858,837,5,919523, 5,925,525, 5,959,098, 5,968,740, 5,981,185, 6,013,440, 6,022,963, 6,027,880, 6,040,138, 6,045,996, and 6,083,697 all of which are incorporated by reference in their entirety for all purposes. In this embodiment, the nucleic acid sample is fragmented and the fragments are exposed to the nucleic acid array. Fragments which contain regions which are complementary to the immobilized probes will hybridize to the array. After hybridization, non-hybridized fragments are removed. The hybridized fragments are then eluted from the array producing a pool of fragments which is enriched for those sequences which are complementary to the immobilized selection probes.

In a preferred embodiment, the vector fragments are bound to magnetic beads. In one particular example of the preferred embodiment, the vector fragments are labeled with biotin and the biotin labeled vector fragments are exposed to strept-avadin coated magnetic beads (available from Dynal, Oslo, Norway and Promega, Madison, Wisconsin). Those target sequences which are bound to the selection probes 170 are then isolated from the nucleic acid population via the separation element 160. Thereafter, isolated target sequences 120 may be separated and isolated from the selection probes 140.

Methods of labeling nucleic acids with biotin will be known to those of skill in the art (see for example, Ausubel et al (Eds.) Current Protocols in Molecular Biology, Sections 3 and 12 in their entirety) and include rTDT-biotin labeling. Briefly, in this procedure the nucleic acid fragments are exposed to biotinylated ddATP. Terminal transferase then transfers the biotin from the ATP molecule to the 3' end of each fragment. Because biotin has a high affinity for strept-avidin, the biotin labeled nucleic acid fragments will bind to the strept-avidin coated magnetic beads. In an alternative preferred embodiment, the vector fragments are attached to beads which are packed into a column, these beads may, for example, be made of polystyrene or any other appropriate support, such as those disclosed above.

Typically, when the separation element comprises some type of solid support, the target-probe complexes are reversibly immobilized to the solid support and the non-hybridized fragments are washed away. This washing must be conducted at a stringency sufficient to remove unbound sequences without removing hybridized sequences. Those of skill in the art will be familiar with appropriate stringency conditions. See for example, Maniatis et al., which is incorporated by reference above.

Isolation of the target sequence from the separation element is typically achieved by denaturing the target-probe complex under the appropriate conditions to release the target sequences and then removing the selection probes by exploiting the properties of the separation element. For example, if the separation element is a solid support, the denatured target sequences are eluted from the solid support. If the separation element is a nucleic acid sequence which itself is bound to another sequence which is itself immobilized to a solid support, care must be taken to elute only the target sequence and not the separation element. This can be done by manipulating the separation element and denaturization conditions such that the separation element has a higher affinity for the immobilized sequence than it has for the target sequence.

In a further embodiment of the presently claimed invention, adapter sequences are added to the ends of the target sequences. Adapter sequences and their use will be well known to those of skill in the art. Typically they are short oligonucleotides of known sequence between 5 and 20 bases in length but can be much longer as desired for a particular application. Adapters are often attached to nucleic acid sequences to act as primers, tags, separation elements or for any desired use. Information regarding the use, methods of attachment, and design of adapter sequences can be found in, for example, Maniatis et al., incorporated by reference above.

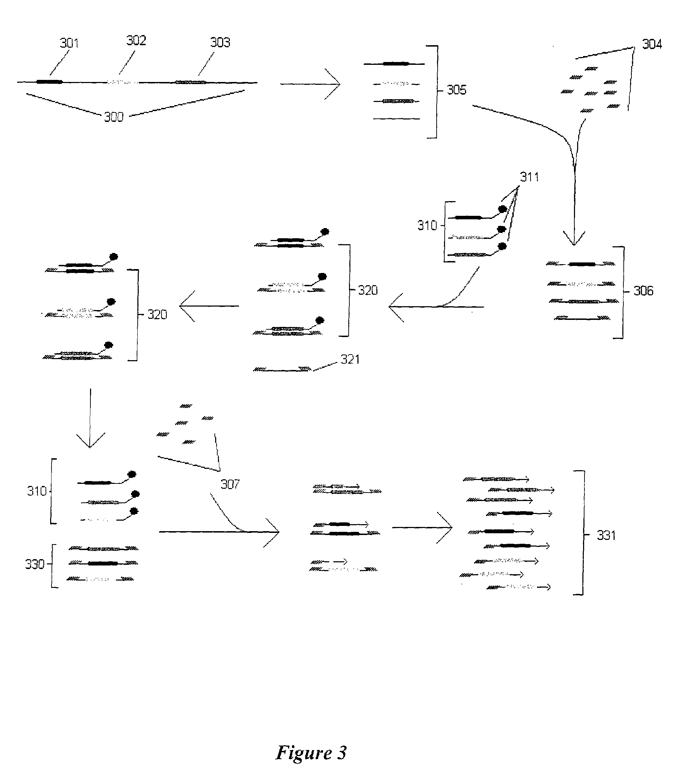

As depicted schematically in Figure 3, adapter sequences may be used to amplify the target fragments after enrichment. A nucleic acid sample 300 is fragmented to produce fragments 305. Adapter sequences 304 are added to the 5' and 3' ends of the fragments producing target sequences 306. Selection probes 310 containing separation elements 311 are exposed to the target sequences under suitable hybridization conditions. The hybridized sequences 320 are isolated from the non-hybridized fragments 321 via the separation element. After isolation, the target sequences are separated from the selection probes producing enriched target sequences 330. Primers 307 which are complementary to the 5' and 3' adapter sequences are added to initiate amplification of the target sequences producing amplified targets 331.

While Figure 3 depicts addition of the adapter sequence prior to exposure to the selection probes, those of skill in the art will appreciate that the adapter sequences may be added at any time during the isolation process. In the embodiment in which the DNA is fragmented with known restriction enzymes, adapters may be designed which will specifically hybridize to the known overhangs produced by the specific restriction enzymes used. Because these adapters may later be used as primer sites for PCR, it may be desirable to design adapters containing sequences which are know to be appropriate PCR priming sequences. Alternatively, if a linear method of amplification is to be used, such as that described in International PCT Application WO 90/06995, one or more of the adapters may also include a promoter sequence.

Alternatively, if methods of fragmentation are employed such that the ends of the fragments are unknown, the ends of the fragments may be filled in with the appropriate nucleotides, for example, by the use of Klenow, and adapters may be blunt-end ligated to the fragments. Methods of filling in DNA overhangs will be known to those of skill in the art. See, for example, Ausubel, et al., (Eds), Current Protocols in Molecular Biology, Section 3.5.9 and throughout. Blunt end hybridization is described in, for example, Ausubel, et al., (Eds) Current Protocols in Molecular Biology (Sections 3.143.2 and 3.16.8). Of course this method may be employed even when the ends of the fragments are known.

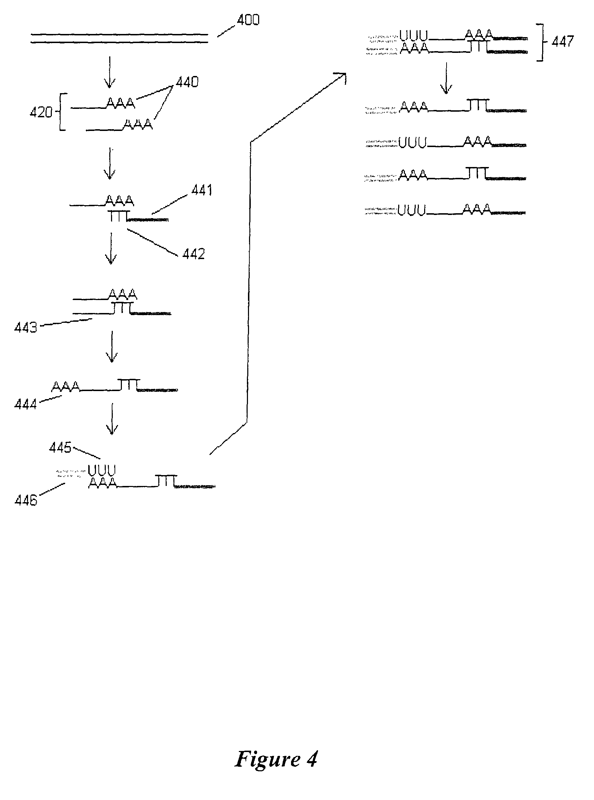

In yet another method, adapter sequences may be attached by employing tailing and extension reactions. In this method, depicted in Figure 4, the complex sample 400 is heat denatured and then fragmented employing any of the means described above. (Steps not shown). Poly A tails 440 are then added to the 3' ends of the fragments 420 by the addition of terminal transferase and dATPs. See for example, Ausubel, et al., (Eds) Current Protocols in Molecular Biology, Section 3.6.2. A primer sequence 442 containing a poly T sequence and a 5' tag sequence 441 is then hybridized to the fragments and extended by polymerase. The resulting double stranded extension product 443 is then denatured and a poly A tail 444 is then added to the single stranded extension product as described above. A second primer sequence 445 containing a poly U sequence and a 5' tag sequence 446 is then hybridized to the resulting sequence and extended. The resulting target sequences 447 comprise a 5' tag sequence, a 5' poly T or U, the original fragment, a 3' poly A, and a 3' tag sequence. The target fragments may then be amplified by adding primers which are complementary to the 5' and 3' tag sequences.

There are many known methods of amplifying nucleic acid sequences including e.g., PCR. See, e.g., *PCR Technology: Principles and Applications for DNA Amplification* (ed. H.A. Erlich, Freeman Press, NY, NY, 1992); *PCR Protocols: A Guide to Methods and Applications* (eds. Innis, et al., Academic Press, San Diego, CA, 1990); Mattila et al., *Nucleic Acids Res.* 19, 4967 (1991); Eckert et al., *PCR Methods and Applications* 1, 17 (1991); *PCR* (eds. McPherson et al., IRL Press, Oxford); and U.S. Patent 4,683,202.

Other suitable amplification methods include the ligase chain reaction (LCR) (e.g., Wu and Wallace, *Genomics* 4, 560 (1989) and Landegren et al., *Science* 241, 1077 (1988)), transcription amplification (Kwoh et al., *Proc. Natl. Acad. Sci. USA* 86, 1173 (1989)), and self-sustained sequence replication (Guatelli et al., *Proc. Nat. Acad. Sci. USA*, 87, 1874 (1990)) and nucleic acid based sequence amplification (NABSA). The latter two amplification methods include isothermal reactions based on isothermal transcription, which produce both single-stranded RNA (ssRNA) and double-stranded DNA (dsDNA) as the amplification products in a ratio of about 30 or 100 to 1, respectively.

As those of skill in the art will appreciate, after isolation and amplification, the resulting sequences may be further analyzed using any known method including sequencing, HPLC, hybridization analysis, etc.

In a preferred embodiment, isolated and amplified sequences are hybridized to probes which are immobilized to a solid support, such as a DNA microarray. The sequences prepared according to the methods of the presently claimed invention are appropriate for both directed arrays and generic "tag" arrays.

In directed arrays, the immobilized probes on the array comprise sequences which are designed to be complementary to the sequences being interrogated (interrogation sequence). Examples of commercially available directed arrays are the Gene Chip® HuSNP™ Probe Array and the Gene Chip® HIV PRT Plus Probe Array (both available from Affymetrix, Inc., Santa Clara, CA). These arrays interrogate specific sequences from human and the HIV virus respectively and enable the investigator to determine whether a given sample contains a sequence which diverges from the normal or wildtype sequences that the array is designed to interrogate.

In generic tag arrays, the immobilized probes are not designed to be complementary to a specific sequence to be interrogated. Rather, the probes are designed to be complementary to a set of tag sequences which can be attached to any sequence to be interrogated. The tag arrays allow for flexibility in the analysis to be performed. An example of a commercially available tag array is the Gene Chip® GenFlex array (Affymetrix, Inc., Santa Clara, CA.) Sequences prepared using the methods described in the currently claimed invention may be particularly well suited for use with a generic tag array since the adapter sequences used to amplify the target sequences may be designed to contain a tag sequence which will hybridize to a probe on the tag array.

Methods of making arrays and their uses are described in, for example, US Patent Nos. 5,143,854, 5,242,979, 5,252,743, 5,324,663, 5,384, 261, 5,405,783, 5,412,087, 5,424,186, 5,445,934, 5,451,683, 5,482,867, 5,489,678, 5,491,074, 5,510,270, 5,527,681, 5,550,215, 5,571,639, 5,593,839, 5,599,695, 5,624,711, 5,631,734, 5,677,195, 5,744,101, 5,744,305, 5,753,788, 5,770,456,5,831,070, 5,856,011, 5,858,695, 5,861,242, 5,871,928, 5,874,219, 5,858,837,5,919523, 5,925,525, 5,959,098, 5,968,740, 5,981,185, 6,013,440, 6,022,963, 6,027,880, 6,040,138, 6,045,996, and 6,083,697 all of which are incorporated by reference in their entirety for all purposes.

### METHODS OF USE

The methods and isolated fragments of the presently claimed invention can be used for a wide variety of applications including genotyping of individuals or populations of individuals. Furthermore, the methods and isolated fragments of the presently claimed invention are particularly well suited for study and characterization of extremely large regions of genomic DNA, such as chromosomal regions.

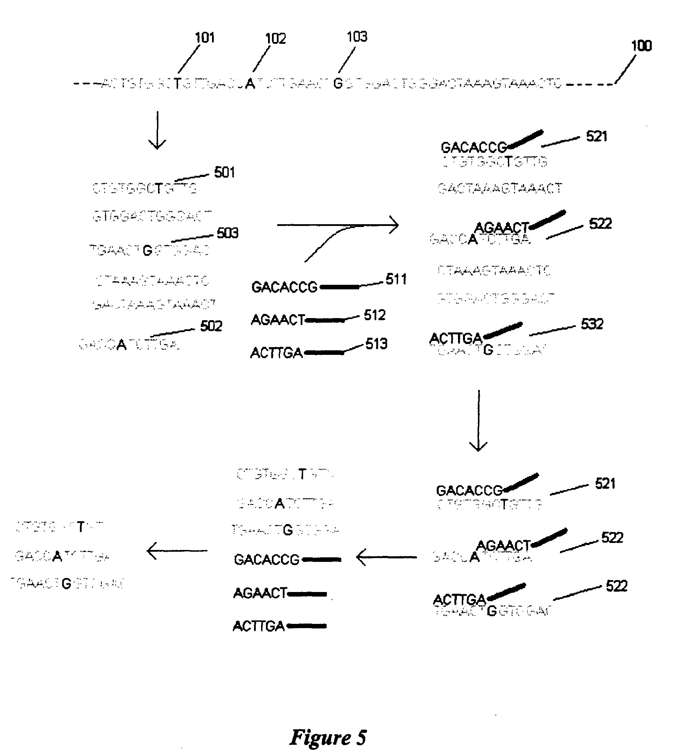

In one method of use, depicted schematically in Figure 5, the methods and isolated fragments of the presently claimed invention are used to genotype an individual. The region (or regions) of interest may be chosen due to a known presence of a polymorphic locus 101, 102, and 103 within the region. DNA samples 100 are obtained from an individual to be genotyped. The DNA is fragmented. The fragments are exposed to selection probes 511, 512, and 513 which are comprised of a complementary segment which is capable of hybridizing to a portion of the region of interest and a separation element under conditions suitable to allow for hybridization between the complementary portions of target sequences and the selection probes. The target-probe complexes 521, 522 and 523 are isolated from the non-targeted sequences via the separation element. Once isolated from the nucleic acid population, the target sequences are then separated and isolated from the selection probes. The target sequences may then be amplified (step not shown) resulting in an isolated and amplified population of target sequences containing the polymorphic sites of interest. This population of target sequences 635 can then be genotyped using any number of known methods including traditional sequencing methods, hybridization to arrays, etc. Microarrays which specifically interrogate for polymorphisms are commercially available. (See, e.g. the GeneChip® HuSNP™ array available from Affymetrix, Inc. Santa Clara, CA.)

The area of complementarity between the target sequence and the selection probe may or may not encompass the polymorphic site. Because the initial nucleic acid sample is fragmented randomly, the selection probe should be complementary to a region adjacent to the polymorphic site so as to ensure that the polymorphic site is included within the fragment. Preferably, selection probes should be within at least 25 bases of the polymorphic site. It should be noted that use of a selection probe which spans the polymorphic site could bias toward one of the polymorphic forms since the exactly complementary sequence will hybridize with a higher affinity than a sequence with a one base mismatch. Various methods may be employed to overcome this. For example, stringency of washing conditions may be manipulated, or a base which will hybridize effectively with either of the polymorphic forms may be substituted in the position where the probe will bind to the target sequence. Alternatively, moving the polymorphic site to one end of the complementary portion will reduce the effect of non-complementarity on affinity. Ideally, a selection of overlapping probes spanning the polymorphic site is used so as to allow for the best capture of fragments containing the regions of interest.

Methods of detecting hybridization on array will be well known to those of skill in the art and may or may not include labeling of the probe, the target, or the probe-target duplex.

In another method of use, the region of interest may be a chromosomal region or an entire chromosome. It is often desirable to isolate and amplify a single chromosome or chromosomal region from a sample of total genomic DNA in order to, for example, simplify experimental design and focus experimental results. In studies attempting to study entire genomes, for example, experiments can be designed to study one chromosome or chromosomal region at a time rather than attempting to study the entire genome in a single experiment. Furthermore, it may be desirable to focus study on a single chromosome which is known or believed to be associated with a certain phenomena.

DNA samples are obtained containing the chromosome or chromosomal region to be isolated. For example, total genomic DNA may be used as the initial sample. The DNA sample is fragmented. Again, any method of fragmentation may be employed as described above. However, in a preferred embodiment, fragments are generated through enzymatic digestion with one or more restriction enzymes to leave known overhangs.

Adapter sequences are added to the 5' and 3' ends of the fragments producing a pool of sequences comprising target sequences and non-target sequences. The pool of sequences is then exposed to selection probes which are comprised of a complementary segment which is capable of hybridizing to the chromosome or chromosomal region to be isolated and a separation element under conditions suitable to allow for hybridization between the complementary portions of target sequences and selection probes. In this case, it may be desirable to use a number of selection probes which in combination span the entire length of the chromosome or chromosomal region , the method depicted in Figure 2, for example may be employed to generate a number of selection probes spanning the entire length of the desired region. The selection probes may be generated by random fragmentation to an average length of between 50 and 150 base pairs or by enzymatic digestion with the same combination of restriction enzymes that were used to fragment the target DNA.

The target-probe complexes are then isolated. As described above, the method of isolation may depend upon the type of solid support to which the vector fragment is attached. For example, if the vector fragments are bound to magnetic beads, the magnetic properties of the beads may be exploited. If, however, the vector fragments are bound to beads which are packed into a column, the genomic DNA is passed through the column and any non-hybridizing fragments will fail to remain in the column. Other methods of isolation will be obvious to those of skill in the art.

The target sequences are then separated and isolated from the selection probes. Typically this will take place by changing the stringency of washing conditions such that the vector and genomic DNA fragments will no longer hybridize. This may include, for example, changing the temperature, pH or other conditions. Again, as an example, if the vector fragments are bound to magnetic beads, the magnetic properties of the beads may be exploited to remove the vector fragments. If the separation element is a bead packed in the column or attached to any solid support, the genomic fragments may be eluted from the column or solid support.

If it is desirable to amplify the isolated fragments, once the genomic fragments containing the desired sequence are isolated, they may be amplified by the polymerase chain reaction (PCR) or another known method using primers complementary to the adapter sequences.

These isolated and amplified chromosomes or chromosomal regions may then be analyzed by any known means, for example, sequencing, genotyping, hybridization analysis, etc.

### EXAMPLES

### A. Enrichment of target fragments using a high-density oligonucleotide array as an affinity reagent.

Samples were prepared according to standard protocols required for the HuSNP™ GeneChip® array (Affymetrix, Inc., Santa Clara, CA) and hybridized to the HuSNP™ array. The image of this initial hybridization was scanned using standard techniques. The array was then washed, hybridized fragments were eluted and the arrays were scanned again to check for residual hybridization. The eluted fragments were re-amplified and hybridized to a second HuSNP™ array.

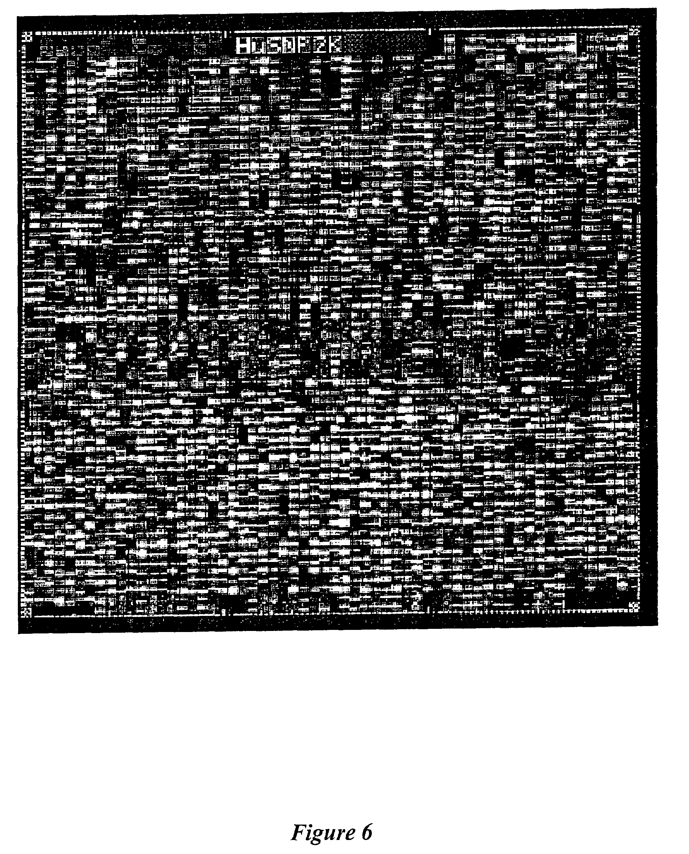

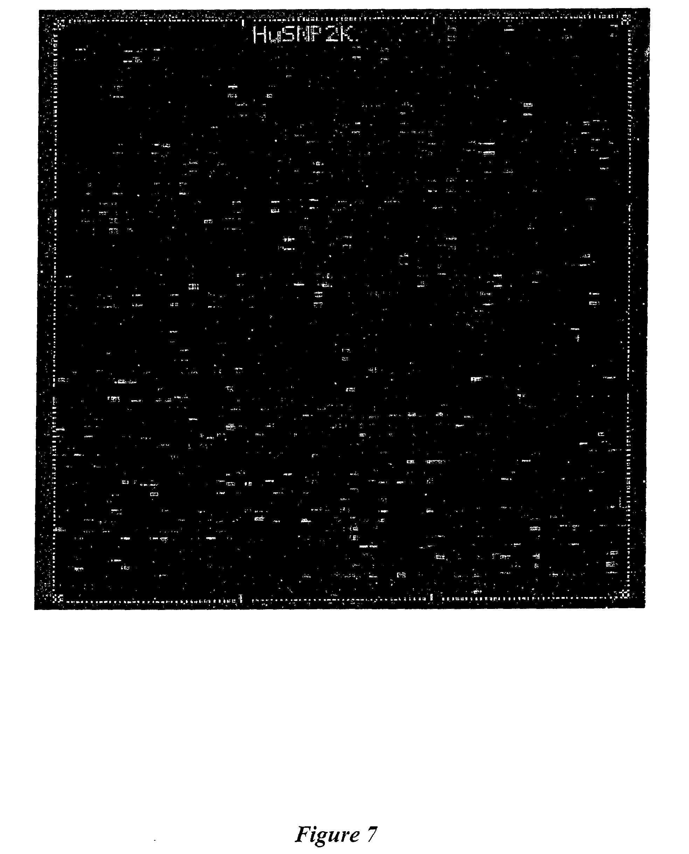

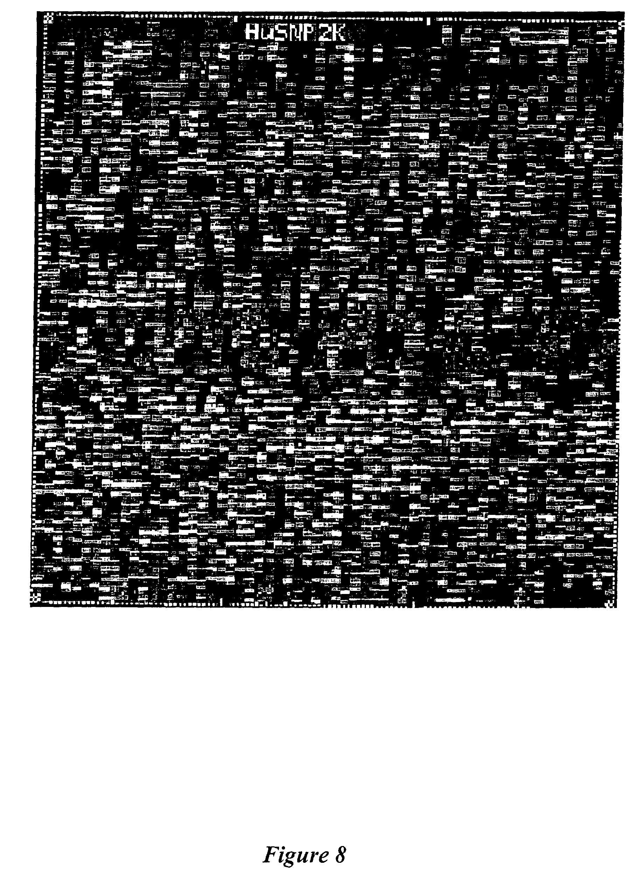

Figure 6 is the scanned image of the initial hybridization. Figure 7 is the scanned image of the first array after elution. Very little signal is seen compared to Figure 6, demonstrating successful elution of the hybridized fragments. Figure 8 is the scanned image of the hybridization of the re-amplified eluted fragments to a second array. Comparison of Figure 8 to Figure 6 shows a much sharper signal with what appears to be significantly less background noise.

### B. Enrichment of small amounts of starting material using an oligonucleotide array as an affinity reagent.

Samples were prepared according to standard protocols required for the HuSNP™ GeneChip® array (Affymetrix, Inc., Santa Clara, CA). The resulting fragments were then diluted to 10¹⁰, 10⁹, 10⁸, 10⁷ and 10⁵ copies (the normal assay produces approximately 10¹¹ copies.) The various dilutions were then hybridized to the HuSNP™ array. The array was washed, and the hybridized fragments were eluted. The eluted fragments were then re-amplified and hybridized to a second HuSNP™ array.

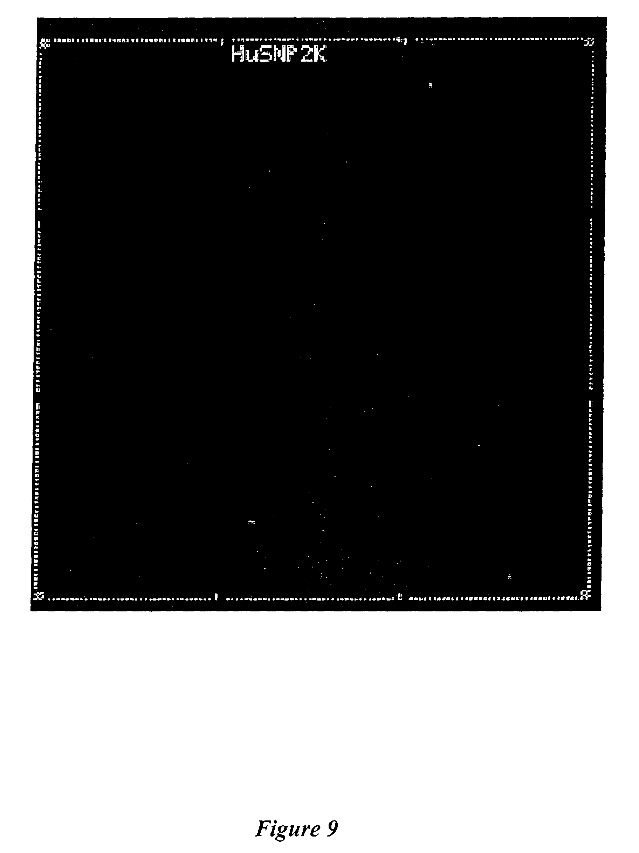

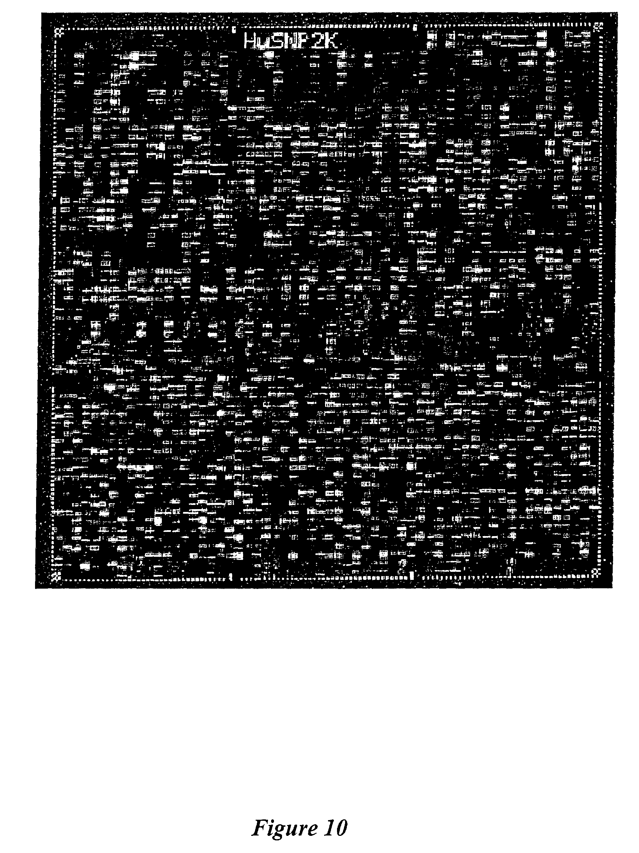

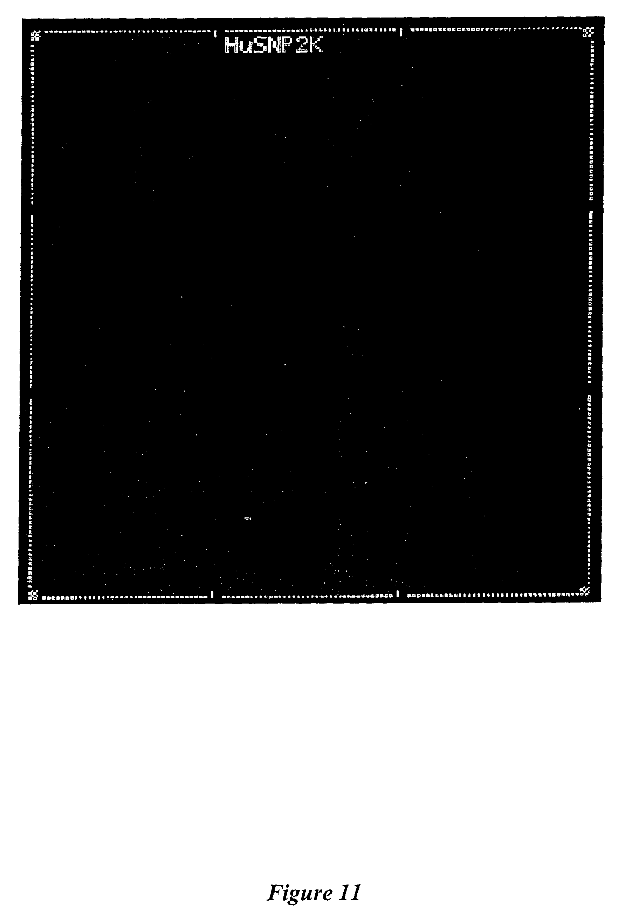

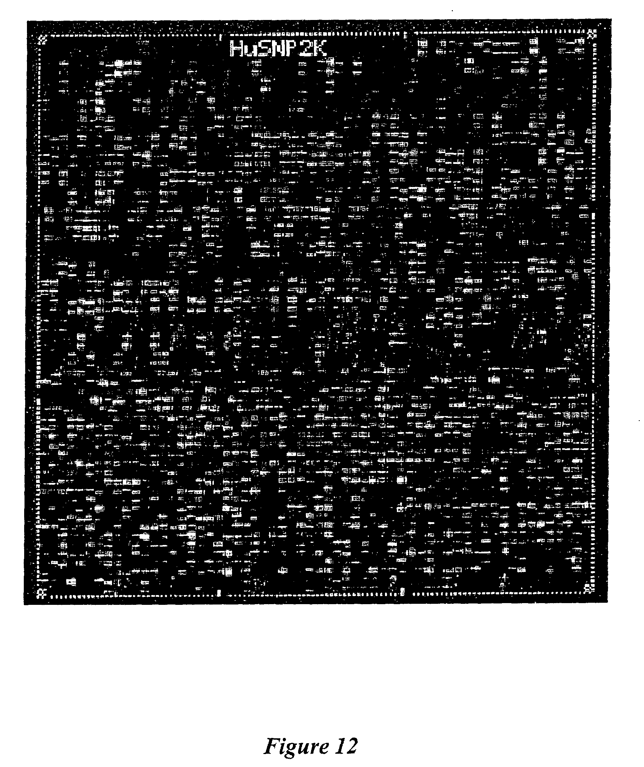

Figure 9 is the scanned image of the initial hybridization of the 10⁷ dilution. Very little signal is present. Figure 10 is the scanned image of the re-amplified eluted fragments from Figure 9 hybridized to the second array. Figure 11 is the scanned image of the initial hybridization of the 10⁵ dilution. Very little signal is present. Figure 12 is the scanned image of the re-amplified eluted fragments from Figure 11 hybridized to the second array. A sharp, clear signal is seen, demonstrating that small amounts of initial material, as little as 10⁵ copies, can be detected using this technique.

### C. Enrichment of specific fragments and generic amplification using beads.

Biotinylated short oligonucleotides were bound to pre-washed streptavidin coated magnetic beads (Dynal, Oslo, Norway). The oligonucleotides were designed to be complementary to one strand of the target fragments with 25 nucleotides. The beads were washed twice with washing solution [5 mM Tris-HCl (pH 7.5), 0.5 mM EDTA and 1 mM NaCl] at room temperature.

Target genomic DNA was boiled with human Cot-1 DNA for 15 min in 10 mM MES buffer (pH 6.5) with 1M NaCl. The mixture was then cooled on ice for 3 min. The mixture was then mixed with the oligonucleotide-bound beads. The target DNA was previously digested with EcoRI and ligated with adapters [5'-d(GATCCGAAGGGGTTCGAATT)-3' (SEQ ID NO: 1) and 5'-d(pGAATTCGAACCCCTTCGGATC)-3' (SEQ ID NO: 2)].

The target DNA was then hybridized to the beads at 50 °C on a rotisserie overnight. The beads were then washed with 50µl washing buffer twice, transferred the solution to a clean new tube, washed for 3 more times with 50 µl washing buffer. We then resuspended the beads in 50µl d. H₂O and incubate at 80 °C for 2 min and then recovered the solution.

We amplified the enriched target with PCR in a 50 µl mixture with 15 mM Tris-HCl (pH 8.0), 50 mM KCl, 2.5 mM MgCl₂, 200 mM dNTPs, 3 mM primer [5'-d(GATCCGAAGGGGTTCGAATT)-3' (SEQ ID NO: 1)], 2 µl enriched target DNA as template and 1 unit AmpliGold polymerase (PE). The PCR was started with 10 min 95 °C incubation followed by 35 cycles of 2 min at 94 °C, 0.5 min at 57 °C, 2min at 72 °C. The mixture was finally incubated for 5 min at 72 °C and kept at 4 °C.

The PCR product was purified with QIAquick PCR Purification kit (Qiagen) according to the manufacturer's instruction. We then fragmented the DNA with DNase I and label with biotin-N⁶-ddATP as follow. In each tube, we incubated 5 µg DNA with 0.3 unit DNaseI (Promega) at 37 °C for 15 min in a 45 µl mixture also containing 10 mM Tris-Acetate (pH 7.5), 10 mM magnesium acetate and 50 mM potassium acetate. The reaction was stopped by heating the sample to 95 °C for 15 min. We then labeled the sample by adding 30 unit terminal transferase and 2 pmol biotin-N⁶-ddATP (Dupont NEN) followed by incubation at 37 °C for 90 min, and heat inactivation at 95 °C for 15 min.

The labeled DNA was hybridized to chips in a hybridization mixture containing 2 µg labeled DNA, 3.5 M tetraethylamonium chloride, 10 mM MES (pH 6.5), 0.01% Triton-100, 20 µg herring sperm DNA, 100 µg bovine serum albumin and 200 pM control oligomer at 44 °C for 40 hours on a rotisserie at 40 rpm. We then washed the chip with 0.1 M NaCl in 10 mM MES for 44 °C for 30 min on a rotisserie at 40 rpm. We first stained with staining solution [ 10 mM MES (pH 6.5), 1 M NaCl, 10 µg/ml streptavidin R-phycoerythrin, 0.5 mg/ml acetylated BSA, 0.01% Triton-100] at 40 °C for 15 min. Then we washed with 6x SSPET [ 0.9 M NaCI, 60 mM NaH₂PO₄ (pH 7.4), 6 mM EDTA , 0.005 % Triton-100] on a fluidics station (Affymetrix) 10 times at 22 °C. We then conducted Anti-streptavidin antibody staining at 40 °C for 30 min with antibody solution [10 mM MES (pH 6.5), 1 M NaCI, 2 mg anti-streptavidin antibody (Vector), 0.5 mg/ml acetylated BSA, 0.01% Triton-100]. We then stained again with staining solution for 15 min followed by 6x SSPET washing as in the previous steps. The chips were scanned with a confocal chip scanner at 560 nm.

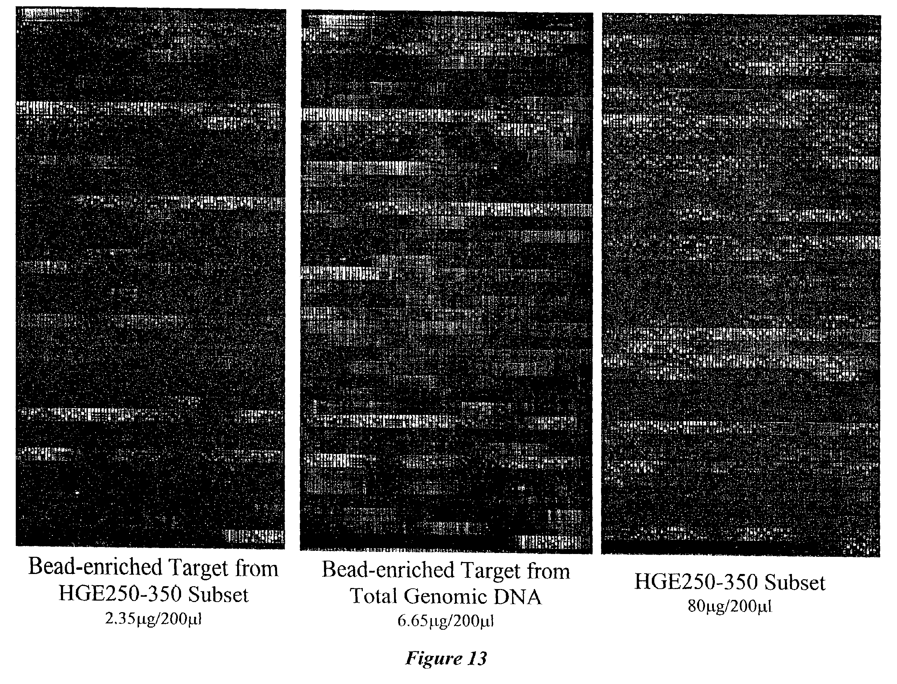

Figure 13 shows the hybridization results.

### D. Enrichment of chromosomal sequences using cloned sequences and generic amplification

We isolated BAC DNA from clone p_M11 (Accession number AC004033) from bacteria. We then fragmented the BAC with restriction enzymes (EcoR I, Sau3A I, Hpa II, Csp6A I, Mse I and Bfa I). The enzymes were then heat inactivated.

We biotinylated 5 µg digested BACs in 50 µl solution by adding 30 unit terminal transferase and 2 pmol biotin-N⁶-ddATP (Dupont NEN) followed by incubation at 37 °C for 90 min, and heat inactivation at 95 °C for 15 min. We removed free biotin-ddATP with Microcon-10 columns.

We bound the BAC DNA to streptavidin-coated magnetic beads by incubating at room temperature for 45 min. The beads were washed with 50 µl washing buffer twice, transferred the solution to a clean new tube, and then washed for 3 more times with 50 µl washing buffer. We resuspended the beads in 50µl 20 mM MES (pH 6.5) with 2 M NaCI.
We then digested 6 µg total human genomic DNA with six restriction enzymes (EcoR I, Sau3A I, Hpa II, Csp6A I, Mse I and Bfa I) separately. Next, we heat inactivated the restriction enzymes.

We then ligated the digested DNA to corresponding adapters.

We then pooled and denatured the ligated genomic DNA in 50 µl final volume. The solution was cooled on ice.

We then mixed the genomic DNA with beads. We hybridized at 50 °C on a rotisserie overnight.

The DNA solution was recovered and washed as described above. We resuspended the beads in 10 µl freshly prepared 0.1 N NaOH and recovered the solution. We then neutralized with 10 µl 1 M Tris-HCI (pH 7.4) and used 1 µl as PCR template for PCR amplification.

We then PCR amplified in a 50 µl final volume with 15 mM Tris-HCl (pH 8.0), 50 mM KCl, 2.5 mM MgCl₂, 200 mM dNTPs, 3 mM primer [5'-d(GATCCGAAGGGGTTCGAATT)-3' (SEQ ID NO: 1)] and 1 unit AmpliGold polymerase (PE). The PCR was started with 10 min 95 °C incubation followed by 35 cycles of 2 min at 94 °C, 0.5 min at 57 °C, 2min at 72 °C. The mixture was finally incubated for 5 min at 72 °C and kept at 4 °C.

We then purified, digested, labeled and hybridized to microarrays as in C above.

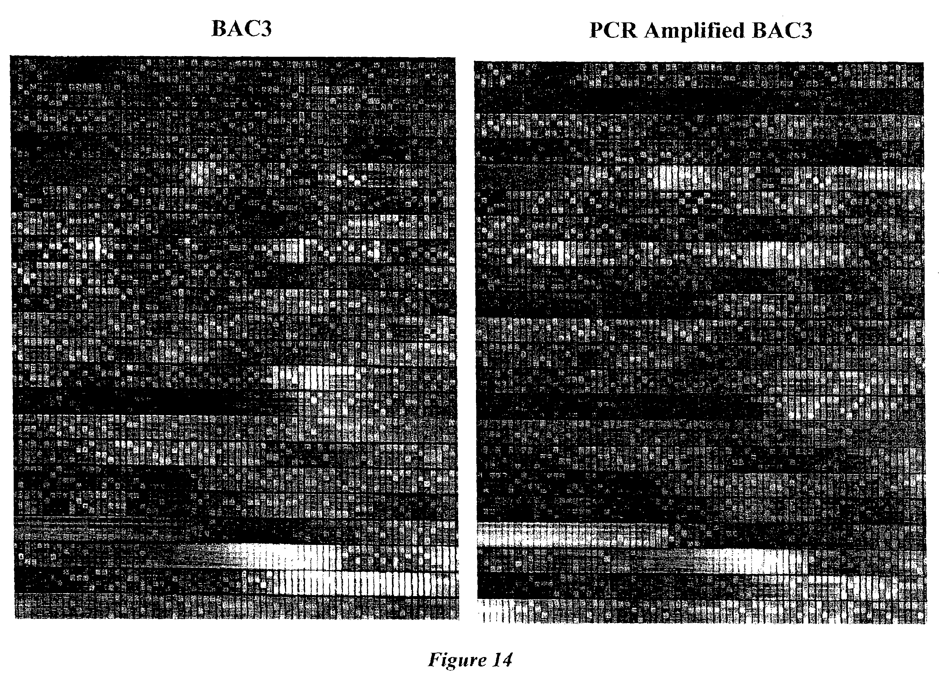

Figure 14 depicts the scanned array image of the enriched DNA before and after amplification.

The presently claimed invention provides greatly improved methods for isolating and amplifying regions of interest in nucleic acid populations. It is to be understood that the above description is intended to be illustrative and not restrictive. Many variations of the invention will be apparent to those of skill in the art upon reviewing the above description. Therefore, it is to be understood that the scope of the invention is not to be limited except as otherwise set forth in the claims.

The presently claimed invention provides methods and kits for isolating and amplifying a specific target sequence from within a nucleic acid population. The presently claimed invention provides selection probes which are complementary to at least a portion of said target sequence and mechanisms for isolating the target-probe complexes from the rest of the nucleic acid population.

## Claims

1. A method of reproducibly obtaining a population of target fragments, wherein each of said target fragments comprises a region of interest, comprising:
fragmenting a nucleic acid population to produce fragments;
exposing said fragments to a plurality of selected probes to form a plurality of target-probe complexes, each of said selection probes comprising a separation element and a region which is complementary to said region of interest;
separating said target-probe complexes from any non-hybridized fragments via said separation element; and
denaturing said target-probe complexes to produce separated target fragments and selection probes.

2. The method of claim 1:
(a) further comprising the step of isolating said target fragments; and optionally wherein said step of isolating comprises removing said selection probes via said separation element;
(b) further comprising the step of amplifying said target fragments; and optionally wherein said step of amplifying comprises:
attaching adapter sequences to the ends of said target fragments; and
adding amplification primers which are complementary to said adapter sequences;
(c) wherein said region of interest comprises a polymorphic locus;
(d) wherein said region of interest is a chromosomal region;
(e) wherein said step of fragmenting comprises exposing said population of nucleic acids to at least one restriction enzyme;
(f) wherein said separation element is a solid support, and optionally said solid support is a bead, and/or said solid support is a streptavadin-coated magnetic bead; and/or said separation element is a nucleic acid sequence; and/or
(g) wherein said selection probes are obtained by:
fragmenting a nucleic acid sequence comprising the complementary sequence to said region of interest; and
attaching a separation element to the resulting fragments; and optionally wherein said nucleic acid sequence is an insert sequence in cloning vector; and further optionally wherein said insert sequence is a chromosomal region.

3. A method of analysing a nucleic acid sample comprising:
fragmenting said nucleic acid population to produce fragments;
exposing said fragments to selection probes to form a plurality of probe-target complexes, each of said selection probes comprising a separation element and a region which is complementary to said region of interest;
separating said probe-target complexes from any non-hybridized fragments via said separation element;
denaturing said probe-target complexes to produce target fragments and selection probes;
amplifying said target fragments; and
hybridizing said amplified target fragments to a nucleic acid array.

4. The method of claim 3, wherein said region of interest either comprises a polymorphic locus, or a chromosomal region.

5. A kit for reproducibly obtaining a population of target fragments wherein each of said target fragments comprises a region of interest comprising:
a population of cells comprising cloning vectors wherein said cloning vectors comprise nucleic acid insert sequences which are complementary to at least a part of said region of interest; and
a plurality of separation elements which are capable of being attached to fragments of said cloning vectors.

6. The kit of claim 5, wherein either said separation elements are beads, and/or the nucleic acid insert sequences comprise chromosomal regions.

7. A method of genotyping an individual comprising:
obtaining a sample of genomic DNA from said individual;
fragmenting said sample to form genomic fragments;
exposing said genomic fragments to selection probes, wherein said selection probes comprise a separation element and a region which is complementary to a region of DNA which is known or believed to be adjacent to a polymorphic site, under suitable conditions to allow for hybridization between said selection probes and said genomic fragments to form a plurality of probe-target complexes;
separating said probe-target complexes from any non-hybridized fragments via said separation element;
denaturing said probe-target complexes to produce target fragments and selection probes;
amplifying said target fragments;
hybridizing said amplified target fragments to an array of probes immobilized to a solid support at known locations, wherein said probes are able to interrogate said polymorphic site; and
detecting the nucleic acid base at said polymorphic site, thus genotyping said individual.

8. An isolated fragment comprising a region of interest obtainable by:
fragmenting a nucleic acid population of interest thereby producing fragments;
exposing said fragments to a selection probe to form a probe-target complex, said selection probe comprising a separation element and a region which is complementary to said region of interest;
separating said probe-target complex from any non-hybridized fragments via said separation element;
denaturing said probe-target complex to produce a target fragment and selection probe; and
isolating said target fragment from said selection probe.

9. A method for reproducibly obtaining target sequences for further analysis comprising:
fragmenting a nucleic acid sample to produce nucleic acid fragments;
exposing said fragments to a plurality of selection probes under suitable condition so as to produce hybridization between complementary sequences, each said plurality of selection probes being designed to be complementary to at least a portion of one of said target sequences, said plurality of selection probes being immobilized to a solid support;
removing any non-hybridized fragments; and
eluting said hybridized fragments to produce a pool of target sequences.

10. The method of claim 9, wherein:
(a) said selection probes are designed to be complementary to a region of said nucleic acid sample which is either known or believed to be polymorphic, or known or believed to be associated with a particular phenotype; and/or
(b) said nucleic acid sample is genomic DNA.

11. An apparatus for reproducibly obtaining a population of target fragments comprising an array of selection probes immobilized to a solid support, wherein each of said selection probes is designed to be complementary to at least a portion of one of said target fragments.


SEQUENCE LISTING

<110> Affymetrix, Inc.

<120> TARGET ENRICHMENT AND AMPLIFICATION FOR
ARRAY ANALYSIS

<130> 2719.2020002

<150> US 60/228,253
<151> 2000-08-26

<150> US 09/766,212
<151> 2001-01-19

<160> 2

<170> FastSEQ for Windows Version 4.0

<210> 1
<211> 20
<212> DNA
<213> Artificial Sequence

<220>
<223> Synthetic oligonucleotide

<400> 1
gatccgaagg ggttcgaatt 20

<210> 2
<211> 21
<212> DNA
<213> Artificial Sequence

<220>
<223> Synthetic oligonucleotide

<400> 2
gaattcgaac cccttcggat c 21